# EUROPÄISCHE PATENTANMELDUNG

(11) **EP 3 567 370 A1**
(43) Veröffentlichungstag der Anmeldung: **13.11.2019**
(21) Anmeldenummer: 18171202.7
(22) Anmeldetag: 08.05.2018
(51) Int. Cl.: G01N 33/20, C21C 5/46

(54) **LANZENSONDE MIT ABGABE VON REFERENZSPANNUNGEN**

(71) Anmelder: Primetals Technologies Austria GmbH, 4031 Linz (AT)
(72) Erfinder: HUSAKOVIC, Adnan, 4150 Rohrbach (AT); HARTL, Franz, 4720 Kallham (AT); ROHRHOFER, Andreas, 4020 Linz (AT); FISCHER, Paul, 4040 Linz (AT); KUEHAS, Thomas, 4225 Luftenberg (AT); MAYRHOFER, Anna, 4722 Bruck-Waasen (AT); VOGLMAYR, Bernhard, 4060 Linz (AT)
(74) Vertreter: Metals@Linz

(57) **Zusammenfassung**

Eine Sonde (1) für eine Lanze (2) eines metallurgischen Gefäßes (3) ist im wesentlichen bezüglich einer Sondenachse (5) rotationssymmetrisch, insbesondere zylindrisch. Die Sonde (1) ist durch Stecken in Richtung der Sondenachse (5) auf die Lanze (2) des metallurgischen Gefäßes (3) aufsteckbar. Die Sonde (1) weist eine Anzahl von Signalkontakten (6) und eine Anzahl von übrigen Kontakten (7) auf, die beim Aufstecken der Sonde (1) auf die Lanze (2) mit jeweiligen Gegenkontakten (8) der Lanze (2) kontaktiert werden. Die Sonde (1) weist eine Referenzschaltung (9) auf, die derart ausgebildet ist, dass sie pro Signalkontakt (6) eine Anzahl von Referenzspannungen (U) generiert und über den jeweiligen Signalkontakt (6) an den jeweiligen Gegenkontakt (8) ausgibt.

## Beschreibung

### Gebiet der Technik

Die vorliegende Erfindung geht aus von einer Sonde für eine Lanze eines metallurgischen Gefäßes,
- wobei die Sonde im wesentlichen bezüglich einer Sondenachse rotationssymmetrisch ist, insbesondere zylindrisch ist,
- wobei die Sonde durch Stecken in Richtung der Sondenachse auf die Lanze des metallurgischen Gefäßes aufsteckbar ist,
- wobei die Sonde eine Anzahl von Signalkontakten und eine Anzahl von übrigen Kontakten aufweist, die beim Aufstecken der Sonde auf die Lanze mit jeweiligen Gegenkontakten der Lanze kontaktiert werden.

Die vorliegende Erfindung geht weiterhin aus von einer Gruppe derartiger Sonden,
- wobei die Sonden den gleichen mechanischen Aufbau aufweisen,
- wobei einer der Signalkontakte der Sonden ein Sonderkontakt ist,
- wobei die Sonderkontakte der Sonden beim Aufstecken der jeweiligen Sonde in die Lanze denselben Gegenkontakt kontaktieren.

### Stand der Technik

Derartige Sonden werden insbesondere in der stahlerzeugenden Industrie verwendet, um mittels in den Sonden integrierter Sensoreinrichtungen Zustandsgrößen einer in dem metallurgischen Gefäßes befindlichen Metallschmelze zu erfassen, beispielsweise deren Temperatur oder deren Sauerstoffaktivität. Weiterhin können mittels derartiger Tauchsonden auch Proben aus der Metallschmelze gezogen werden, welche später in einem Labor analysiert werden. Die Sonden sind in der Regel Einweg-Tauchsonden. Es wird jeweils eine Sonde auf die Lanze aufgesteckt. Danach erfolgt der Messvorgang. Die erfassten Zustandsgrößen bzw. die hiermit korrespondierenden Messsignale werden über die zugehörigen Signalkontakte der Sonde und die zugehörigen Gegenkontakte der Lanze und weiter über eine Verkabelung der Lanze zu einer Auswertungseinrichtung geführt. Dort werden sie automatisch oder durch eine Bedienperson ausgewertet. Nach dem Messvorgang wird die jeweilige Sonde von der Lanze abgezogen. Dieser Vorgang kann nach Bedarf manuell oder automatisch erfolgen. Danach wird die jeweilige Sonde weggeworfen. Dies ist erforderlich, weil insbesondere die Sensoreinrichtungen der jeweiligen Sonde durch die große Hitze der Metallschmelze zerstört werden. Soweit erforderlich, wird vor dem Wegwerfen die gezogene Probe entnommen.

Um Messsignale zu liefern, welche möglichst genau mit der jeweils erfassten tatsächlichen Zustandsgröße korrespondieren, muss zunächst die jeweilige Sensoreinrichtung als solche fehlerfrei arbeiten. Dies wird durch die Hersteller der Sonden aufgrund einer entsprechenden Qualitätskontrolle gewährleistet. Weiterhin dürfen die Messsignale, die sich oftmals im mV-Bereich bewegen, auf dem Weg zur Auswertungseinrichtung nicht verfälscht werden. Mögliche Quellen für eine Signalverfälschung sind insbesondere Übergangswiderstände von den Signalkontakten und/oder den übrigen Kontakten zu den jeweils zugehörigen Gegenkontakten. Weiterhin ist eine Signalverfälschung auch durch die Verkabelung zwischen den Gegenkontakten der Lanze und der Auswertungseinrichtung möglich. Insbesondere eine mangelhafte thermische Isolierung der Verkabelung kann zu Messfehlern führen.

Die Einweg-Tauchsonden des Standes der Technik werden aus gewickeltem Karton gefertigt. Der Karton verkohlt während des Messvorgangs, insbesondere beim Eintauchen in die Metallschmelze. Insbesondere aufgrund dieser Verkohlung können sich beispielsweise Verbrennungsrückstände der Sonden an den Gegenkontakten ablagern. Derartige Ablagerungen können zu deutlichen Änderungen der Übergangswiderstände zwischen den Signalkontakten/übrigen Kontakten einerseits und den Gegenkontakten andererseits führen.

Verfälschte Messergebnisse führen, sofern sie fälschlicherweise verwertet werden, zu einer falschen Prozessführung mit allen damit verbundenen Folgen wie beispielsweise Verzögerungen in der Produktion, dem Einsatz von nicht erforderlichen Zuschlagstoffen oder Energieträgern, einer Änderung des Produktionsplans oder Qualitätseinbußen.

Um die Gegenkontakte der Lanze und die Verkabelung der Lanze auf einwandfreie Funktion zu überprüfen, existieren spezielle Testgeräte. Derartige Testgeräte sind sogenannte Handheld-Geräte, die über ein Kabel mit einem Adapter verbunden sind. Der Adapter ist relativ klein. Typischerweise weist der Adapter einen Durchmesser von ca. 3 cm und eine Länge von ca. 7 bis ca. 10 cm auf. Der Adapter kann mit den Gegenkontakten der Lanze verbunden werden. Nachdem der Adapter mit den Gegenkontakten verbunden wurde, betätigt eine Bedienperson Betätigungselemente des Testgeräts, so dass das Testgerät festgelegte Referenzspannungen abgibt und dadurch entsprechende Messsignale simuliert. Werden die festgelegten Referenzspannungen korrekt an die Auswertungseinrichtung übermittelt, kann auf einen fehlerfreien Messkreis geschlossen werden. Erfolgt die Übermittlung fehlerhaft, muss die Bedienperson die Gegenkontakte reinigen und eine neuerliche Testmessung vornehmen. Wenn auch dies nicht zum Erfolg führt, muss ein Kontaktstück der Lanze, welches die Gegenkontakte enthält, vor Ort ausgetauscht werden.

Die Verwendung derartiger Testgeräte ist nur während einer Produktionspause des metallurgischen Prozesses möglich. Weiterhin muss sich die Bedienperson zur Durchführung des Tests in den Gefahrenbereich der Anlage begeben, welche das metallurgische Gefäß enthält. Die Bedienperson ist daher einem erhöhten Sicherheitsrisiko ausgesetzt. Aufgrund dieses erhöhten Risikos werden derartige Tests meist nicht in regelmäßigen Abständen durchgeführt, sondern erst dann, wenn aufgrund anderer Umstände der Verdacht vorliegt, dass nicht ordnungsgemäße Messungen vorliegen.

Es sind auch schon vollautomatisch arbeitende Testsysteme bekannt. Bei derartigen Testsystemen ist ebenfalls ein Adapter über ein Kabel mit dem eigentlichen Testgerät verbunden. Der Unterschied zur obenstehend erläuterten Vorgehensweise besteht im wesentlichen darin, dass sowohl das Verbinden des Adapters mit der Lanze als auch das Lösen des Adapters von der Lanze mittels eines Roboters erfolgen und dass das Testgerät insoweit in die Automatisierungstechnik eingebunden ist, dass sowohl das Ausgeben der Referenzspannungen automatisch angestoßen wird als auch der Auswertungseinrichtung automatisch mitgeteilt wird, wann eine Testmessung erfolgt.

### Zusammenfassung der Erfindung

Die Aufgabe der vorliegenden Erfindung besteht darin, Möglichkeiten zu schaffen, mittels derer auf einfache und zuverlässige Weise und insbesondere auch im laufenden Betrieb des metallurgischen Prozesses eine Überprüfung der Messkreise auf Fehlerfreiheit möglich ist.

Die Aufgabe wird durch eine Sonde mit den Merkmalen des Anspruchs 1 gelöst. Vorteilhafte Ausgestaltungen der Sonde sind Gegenstand der abhängigen Ansprüche 2 bis 11.

Erfindungsgemäß wird eine Sonde der eingangs genannten Art dadurch ausgestaltet,
- dass die Sonde eine Referenzschaltung aufweist, die derart ausgebildet ist, dass sie pro Signalkontakt eine Anzahl von Referenzspannungen generiert und über den jeweiligen Signalkontakt an den jeweiligen Gegenkontakt ausgibt, und
- dass, bezogen auf den jeweiligen Signalkontakt, entweder die Anzahl von Referenzspannungen gleich eins ist oder die Referenzspannungen eine Sequenz von nacheinander ausgegebenen Referenzspannungen bilden.

Durch die erfindungsgemäße Ausgestaltung der Sonde ist es insbesondere möglich, die Sonde geometrisch in der gleichen Form wie die konventionellen Sonden auszubilden und damit auch auf die gleiche Art und Weise - beispielsweise mittels eines Roboters - zu handhaben. Insbesondere ist es damit möglich eine derartige Sonde auch während des metallurgischen Prozesses jederzeit zu verwenden.

Im Falle der Abgabe nur einer einzelnen Referenzspannung kann der abgegebene Spannungswert zur Überprüfung des Messkreises (ok/nicht ok) oder zur quantitativen Ermittlung eines Messkreisfehlers verwendet werden. Im letztgenannten Fall kann aus einem etwaigen Messkreisfehler ein Offset ermittelt werden, der bei der späteren tatsächlichen Messung berücksichtigt werden kann. Im Falle der Abgabe einer Sequenz von Referenzspannungen ist es insbesondere möglich, ein Stützstellenfeld zu ermitteln, welches die jeweilige Referenzspannung mit dem jeweils gemessenen Wert verknüpft, und dieses Stützstellenfeld zur späteren Interpolation eines "echten" Messwerts im Rahmen der späteren tatsächlichen Messung zu berücksichtigen.

Die jeweilige Referenzspannung ist eine Gleichspannung. Sie wird für einen Zeitraum gehalten, der erheblich größer als der Zeitraum zum Zuschalten und/oder Abschalten der Referenzspannung bzw. - im Falle einer Sequenz von Referenzspannungen - zum Umschalten von einer Referenzspannung zur nächsten Referenzspannung ist. Insbesondere ist der Zeitraum mindestens fünfmal so groß wie der Zeitraum zum Zuschalten und/oder Abschalten der Referenzspannung bzw. zum Umschalten von einer Referenzspannung zur nächsten Referenzspannung, vorzugsweise mindestens zehnmal so groß.

Zur Generierung der Referenzspannung bzw. der Referenzspannungen gibt es verschiedene Möglichkeiten. Am einfachsten kann dies dadurch realisiert werden, dass die Referenzschaltung einen Spannungsgenerator aufweist, der eine Basisspannung generiert, und die Referenzspannungen aus der Basisspannung über Spannungsteiler generiert werden. Spannungsgeneratoren, die mit hinreichender Genauigkeit eine Basisspannung generieren, sind als solche allgemein bekannt. Es kann rein beispielhaft auf die integrierten Schaltkreise der Baureihe REF 32xx der Firma Texas Instruments (mit xx = 12, 20, 25, 30, 33, 40) verwiesen werden.

Die Spannungsteiler können nach Bedarf als passive oder als aktive Spannungsteiler ausgebildet sein. Passive Spannungsteiler bestehen ausschließlich aus passiven, nicht verstärkenden Elementen, in der Regel ausschließlich aus Widerständen. Sie sind besonders einfach zu realisieren. Aktive Spannungsteiler umfassen Verstärkerschaltungen. Sie weisen den Vorteil auf, dass eine etwaige Begrenzung einer Stromtragfähigkeit des Spannungsgenerators unkritisch ist und unter Umständen auch eine Referenzspannung generiert werden kann, die größer als die Basisspannung des Spannungsgenerators ist.

Die Spannungsteiler können alternativ fest eingestellt oder einstellbar sein. Beispielsweise können im Falle eines passiven Spannungsteilers alternativ nicht veränderliche Widerstände verwendet werden oder es kann mindestens ein veränderlicher Widerstand verwendet werden, beispielsweise ein Potentiometer.

Vorzugsweise sind die Spannungsteiler als temperaturkompensierte Spannungsteiler ausgebildet. Dadurch sind die abgegebenen Referenzspannungen unabhängig bzw. zumindest nahezu unabhängig von der Umgebungstemperatur.

Es ist möglich, dass die Sonde ausschließlich die Referenzspannungen generiert und über die Signalkontakte an die Gegenkontakte ausgibt. Vorzugsweise aber ist die Sonde derart ausgestaltet,
- dass die Sonde eine Anzahl von Sensoreinrichtungen aufweist, mittels derer jeweils eine Zustandsgröße einer in dem metallurgischen Gefäß befindlichen Metallschmelze erfassbar ist und ein mit der jeweiligen Zustandsgröße korrespondierendes Messsignal ausgebbar ist, und
- dass die Sonde eine Umschalteinrichtung aufweist, welche in einem ersten Schaltzustand die Referenzschaltung und in einem zweiten Schaltzustand anstelle der Referenzschaltung die Sensoreinrichtungen an die Signalkontakte anschaltet.

Wenn die Sonde derart ausgebildet ist, kann im laufenden Betrieb des metallurgischen Prozesses vor jeder "echten" Messung mittels der Sensoreinrichtungen der Sonde zunächst eine vorherige Überprüfung der zugehörigen Messkreise erfolgen. Das Ergebnis der Überprüfung kann, wie bereits erwähnt, zur Ermittlung eines Offsets und/oder eines Stützstellenfeldes verwendet werden.

Es ist möglich, dass die Umschalteinrichtung eine Betätigungseinrichtung aufweist, mittels derer die Umschalteinrichtung von einer Bedienperson manuell in den ersten und den zweiten Schaltzustand gebracht werden kann. In diesem Fall kann die Betätigungseinrichtung beispielsweise als (mechanischer oder anderweitig ausgebildeter) Taster ausgebildet sein, so dass durch ein Betätigen des Tasters die Umschalteinrichtung in den ersten Schaltzustand überführt wird, während sie sich bei unbetätigtem Taster im zweiten Schaltzustand befindet. Alternativ kann die Betätigungseinrichtung beispielsweise ein (mechanischer oder anderweitig ausgebildeter) Schalter sein, der bistabil zwischen dem ersten und dem zweiten Schaltzustand hin und her schaltet.

Alternativ zum Vorhandensein einer Betätigungseinrichtung ist es möglich, dass die Umschalteinrichtung einen Signaleingang aufweist, der mit einem der übrigen Kontakte verbunden ist. In diesem Fall kann die Umschalteinrichtung mittels eines Umschaltsignals, das diesem übrigen Kontakt über den korrespondierenden Gegenkontakt zugeführt wird, in den ersten und den zweiten Schaltzustand gebracht werden. Es kann also ein ferngesteuerte Umschalten zwischen dem ersten und dem zweiten Schaltzustand erfolgen.

Wiederum alternativ ist es möglich, dass die Umschalteinrichtung derart ausgebildet ist, dass sie beim Aufstecken der Sonde auf die Lanze stets zunächst den ersten Schaltzustand annimmt und erst später in den zweiten Schaltzustand übergeht. Der spätere Zeitpunkt kann auf verschiedene Art und Weise bestimmt sein.
- Ein Beispiel ist der Ablauf einer vorbestimmten Zeitspanne. Beispielsweise kann die Sonde einen Timer aufweisen, der beim Aufstecken der Sonde auf die Lanze gestartet wird und nach einer vorbestimmten Zeit abläuft. Das Starten des Timers kann beispielsweise dadurch bewirkt werden, dass die Sonde über die Lanze mit elektrischer Energie versorgt wird.
- Ein weiteres Beispiel besteht darin, dass über die Energieversorgung der Sonde beispielsweise ein Kondensator über einen Widerstand allmählich aufgeladen wird und die Umschalteinrichtung vom ersten in den zweiten Schaltzustand wechselt, wenn die über dem Kondensator abfallende Spannung einen Grenzwert erreicht bzw. übersteigt.
- Im Falle des Ausgebens einer Sequenz von Referenzspannungen ist es möglich, dass die Sonde eigenständig überprüft, ob das Ausgeben der Sequenz von Referenzspannungen beendet ist, und danach in den zweiten Schaltzustand übergeht.
- Ein weiteres Beispiel besteht darin, dass die Umschalteinrichtung aufgrund der Vorgabe eines Umschaltsignals über einen der übrigen Kontakte in den zweiten Schaltzustand übergeht. Der Unterschied zu der zuvor erläuterten Vorgehensweise, bei der ein ferngesteuerte Umschalten zwischen dem ersten und dem zweiten Schaltzustand erfolgen kann, kann in diesem Fall darin bestehen, dass nur einmal vom ersten in den zweiten Schaltzustand übergegangen werden kann, danach aber der zweite Schaltzustand unveränderlich beibehalten wird.

Vorzugsweise weist die Sonde eine Zusatzschaltung auf, welche die Umschalteinrichtung ansteuert. Die Zusatzschaltung kann nach Bedarf aus diskreten Bauelementen (Spulen, Kondensatoren, Widerständen, eventuell zusätzlich Dioden und/oder Transistoren) realisiert sein. Alternativ ist es möglich, dass die Zusatzschaltung mit integrierten Schaltkreisen realisiert ist. In analoger Weise ist es möglich, die Referenzschaltung mit diskreten Bauelementen und/oder integrierten Schaltkreisen zu realisieren.

Die Umschalteinrichtung kann nach Bedarf als elektromechanische Umschalteinrichtung oder als elektronische Umschalteinrichtung ausgebildet sein. Im Falle einer elektromechanischen Implementierung werden vorzugsweise Relais verwendet, im Falle einer elektronischen Implementierung Halbleiterschaltelemente, beispielsweise MOSFETs.

In einer besonders bevorzugten Ausgestaltung weist die Sonde einen Hohlraum auf, mittels dessen aus einer in dem metallurgischen Gefäß befindlichen Metallschmelze eine Probe entnommen werden kann. Dadurch ist eine Probenentnahme möglich.

Die Mantelfläche der Sonde besteht vorzugsweise aus Karton, insbesondere aus gewickeltem Karton. Dadurch kann die Sonde auf sehr kostengünstige Weise hergestellt werden. Die Mantelfläche der Sonde kann nicht nur bei einer Sonde, welche zusätzlich zur Referenzschaltung eine Anzahl von Sensoreinrichtungen und eine Umschalteinrichtung aufweist, aus Karton bestehen. Die gleiche Ausgestaltung ist auch möglich, wenn die Sonde ausschließlich die Referenzschaltung aufweist, also nicht auch die Sensoreinrichtungen und die Umschalteinrichtung. Denn auch in diesem Fall kann die Sonde so günstig hergestellt werden, dass sie ohne nennenswerte wirtschaftliche Kosten als Einwegsonde verwendet werden kann. Im Falle einer reinen Referenzsonde - also einer Sonde ohne Sensoreinrichtungen und ohne Umschalteinrichtung - ist aber auch eine Wiederverwendung der Sonde möglich.

Die Aufgabe wird weiterhin durch eine Gruppe von Sonden mit den Merkmalen des Anspruchs 12 gelöst. Eine vorteilhafte Ausgestaltung der Gruppe von Sonden ist Gegenstand des abhängigen Anspruchs 13.

Erfindungsgemäß wird eine Gruppe von Sonden der eingangs genannten Art dadurch ausgestaltet,
- dass die Sonden sich dadurch unterscheiden,
   -- ob und gegebenenfalls in welchem Umfang sie weitere Signalkontakte aufweisen, über die sie jeweils eine Anzahl von Referenzspannungen an den jeweiligen Gegenkontakt ausgeben, und/oder
   -- ob sie eine Sensoreinrichtung und eine Umschalteinrichtung aufweisen, so dass sie über den Sonderkontakt alternativ zu der Anzahl von Referenzspannungen ein Messsignal ausgeben können, und/oder
   -- ob und gegebenenfalls in welchem Umfang sie weitere Sensoreinrichtungen aufweisen und über die Umschalteinrichtung alternativ zu der Anzahl von Referenzspannungen weitere Messsignale ausgeben können und/oder
   -- gegebenenfalls welche Sensoreinrichtungen sie aufweisen und mit welchen Zustandsgrößen der in dem metallurgischen Gefäß befindlichen Metallschmelze die zugehörigen Messsignale korrespondieren und/oder
   -- ob die Sonde einen Hohlraum aufweist, mittels dessen aus der in dem metallurgischen Gefäß befindlichen Metallschmelze eine Probe entnommen werden kann, und
- dass die Sonden sich weiterhin dadurch unterscheiden, dass der Wert der Referenzspannung und/oder die Abfolge der Referenzspannungen, welche die Sonden über den Sonderkontakt ausgeben, voneinander verschieden sind.

Dadurch kann im Rahmen der Referenzmessung, wenn die Sonde also über ihre Signalkontakte und die zugehörigen Gegenkontakte der Lanze die Referenzspannungen ausgibt, anhand der über den Sonderkontakt ausgegebenen Referenzspannung bzw. Referenzspannungen erkannt werden, um welchen Typ von Sonde es sich handelt.

Der Sonderkontakt ist vorzugsweise dadurch bestimmt, dass bei allen Sonden, bei denen über den Sonderkontakt ein Messsignal übertragen werden kann, die zugehörige Sensoreinrichtung eine Temperaturmesseinrichtung ist. Denn unabhängig von der Art von verwendeten Sonden erfassen die Sonden in der Praxis zumindest die Temperatur der Metallschmelze und geben den entsprechenden Messwert über einen ganz bestimmten Signalkontakt, nämlich den Sonderkontakt, und den entsprechenden Gegenkontakt aus.

### Kurze Beschreibung der Zeichnungen

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:
- FIG 1: eine Sonde in einer Seitenansicht
- FIG 2: eine Sonde beim Aufstecken auf eine Lanze,
- FIG 3: eine Sonde, eine Lanze und ein metallurgisches Gefäß,
- FIG 4: ein Schaltbild,
- FIG 5: über einen Signalkontakt ausgegebene Referenzspannungen,
- FIG 6: einen Teil einer Referenzschaltung,
- FIG 7: eine alternative Ausgestaltung eines Teils einer Referenzschaltung,
- FIG 8: eine Modifikation von FIG 6,
- FIG 9: ein weiteres Schaltbild,
- FIG 10: eine Modifikation von FIG 9,
- FIG 11: eine Umschalteinrichtung und eine Zusatzschaltung,
- FIG 12: eine Umschalteinrichtung und eine Referenzschaltung,
- FIG 13: ein weiteres Schaltbild,
- FIG 14: ein Spannungsdiagramm und
- FIG 15: ein Zeitdiagramm.

### Beschreibung der Ausführungsformen

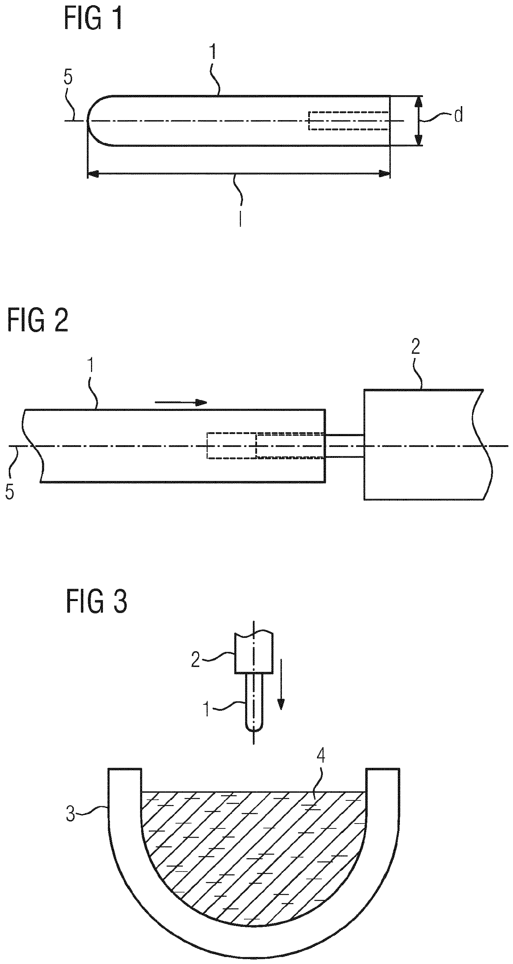

Gemäß FIG 1 ist eine Sonde 1 für eine Lanze 2 (siehe FIG 2) eines metallurgischen Gefäßes 3 (siehe FIG 3) als langgestreckter, im wesentlichen zylindrischer Körper ausgebildet. Im vorderen Bereich, der bei der Verwendung der Sonde 1 in eine Metallschmelze 4 eingetaucht wird, die sich in dem metallurgischen Gefäß 3 befindet, kann die Sonde 1 entsprechend der Darstellung in den FIG 1 bis 3 abgerundet sein. Die Sonde 1 ist also im wesentlichen bezüglich einer Sondenachse 5 rotationssymmetrisch. Eine Länge 1 der Sonde 1 kann im Bereich zwischen 50 cm und 250 cm liegen, insbesondere zwischen 75 cm und 200 cm. Ein Durchmesser d der Sonde 1 ist erheblich geringer. Er liegt meist zwischen 5 cm und 15 cm, insbesondere zwischen 7 cm und 12 cm.

Die Sonde 1 kann, wie in FIG 2 durch einen Pfeil angedeutet ist, durch Stecken in Richtung der Sondenachse 5 auf die Lanze 2 des metallurgischen Gefäßes 3 aufgesteckt werden. In diesem Zustand ist die Sonde 1 mechanisch mit der Lanze 2 verbunden, so dass die Sonde 1 beim Bewegen der Lanze 2 mit bewegt wird. Insbesondere ist es, wie in FIG 3 durch einen Pfeil angedeutet ist, möglich, durch entsprechendes Bewegen der Lanze 2 die Sonde 1 in die Metallschmelze 4 einzutauchen, welche sich in dem metallurgischen Gefäß 3 befindet. Bei der Metallschmelze 4 kann es sich beispielsweise um eine Stahlschmelze handeln.

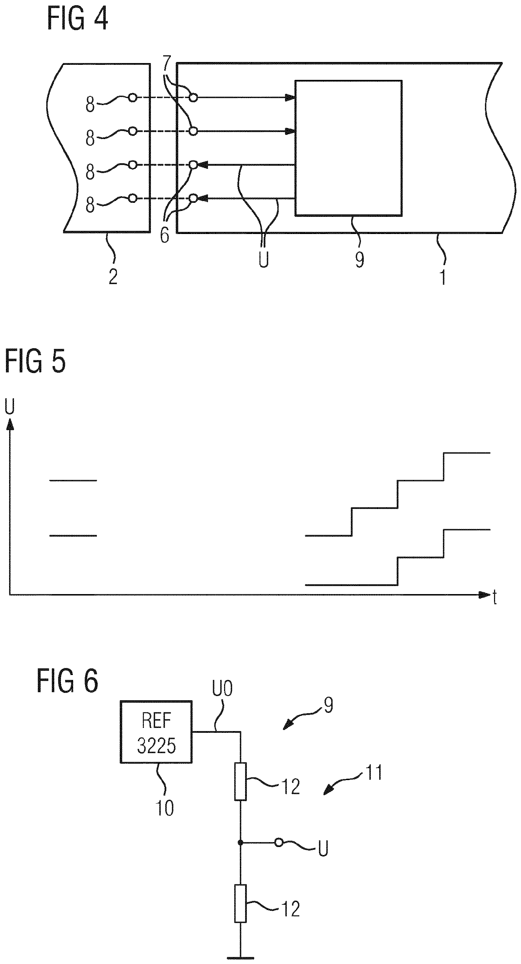

Die Sonde 1 weist entsprechend der Darstellung in FIG 4 eine Anzahl von Signalkontakten 6 auf. Weiterhin weist die Sonde 1 Anzahl von übrigen Kontakten 7 auf. Sowohl die Signalkontakte 6 als auch die übrigen Kontakte 7 werden beim Aufstecken der Sonde 1 auf die Lanze 2 mit jeweiligen Gegenkontakten 8 der Lanze 2 kontaktiert. Von dort werden sie über eine in der Lanze 2 verlaufende, nicht dargestellte Verkabelung einer ebenfalls nicht dargestellten Auswertungseinrichtung zugeführt Die Anzahl an Signalkontakten 6 beträgt minimal eins. Ebenso beträgt die Anzahl an übrigen Kontakten minimal eins. Oftmals sind jedoch mehr als ein Signalkontakt 6 und/oder mehr als ein übriger Kontakt 7 vorhanden. Beispielsweise können zwei übrige Kontakte 7 für eine elektrische Energieversorgung der Sonde 1 und ein oder zwei Signalkontakte 6 zur Übermittlung von Spannungswerten an die Lanze 2 vorhanden sein. Es sind aber auch andere Anzahlen möglich.

Soweit bisher erläutert, entspricht der Aufbau der Sonde 1 dem selben Aufbau, wie er auch im Stand der Technik für derartige Sonden 1 üblich ist. Erfindungsgemäß weist die Sonde 1 jedoch eine Referenzschaltung 9 auf. Die Referenzschaltung 9 ist derart ausgebildet, dass sie pro Signalkontakt 6 eine Anzahl von Referenzspannungen U generiert und über den jeweiligen Signalkontakt 6 an den jeweiligen Gegenkontakt 8 ausgibt. Es ist - siehe in FIG 5 die linke Darstellung - möglich, dass die Referenzschaltung 9, bezogen auf den jeweiligen Signalkontakt 6, nur eine einzige Referenzspannung U generiert. Die Darstellung von zwei Referenzspannungen U ist gewählt, weil beispielsweise auch zwei Signalkontakte 6 vorhanden sein können. Alternativ - siehe in FIG 5 die rechte Darstellung - ist es möglich, dass die Referenzschaltung 9, bezogen auf den jeweiligen Signalkontakt 6, als Funktion der Zeit t mehrere Referenzspannungen U generiert. In diesem Fall bilden die Referenzspannungen U eine Sequenz von nacheinander ausgegebenen Referenzspannungen U. Der Wert der Referenzspannung U bzw. die Werte der Referenzspannungen U und gegebenenfalls auch die Anzahl an Referenzspannungen U der Sequenzen können nach Bedarf bestimmt sein.

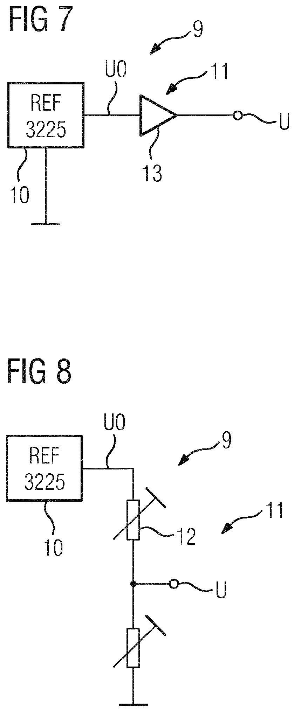

Zum Generieren der Referenzspannung U bzw. der Referenzspannungen U kann die Referenzschaltung 9 entsprechend der Darstellung in den FIG 6 und 7 beispielsweise einen Spannungsgenerator 10 aufweisen, der eine feste Basisspannung U0 generiert. In seltenen Einzelfällen kann die Basisspannung U0 mit der gewünschten Referenzspannung U übereinstimmen. In aller Regel ist sie jedoch von der gewünschten Referenzspannung U verschieden. In diesem Fall kann die Referenzschaltung 9 einen Spannungsteiler 11 aufweisen, mittels dessen die gewünschte Referenzspannung U aus der Basisspannung U0 generiert wird.

Der Spannungsteiler 11 kann entsprechend der Darstellung in FIG 6 als passiver Spannungsteiler ausgebildet sein. In diesem Fall weist der Spannungsteiler 11 ausschließlich passive Elemente auf, insbesondere Widerstände 12. Alternativ kann der Spannungsteiler 11 entsprechend der Darstellung in FIG 7 als aktiver Spannungsteiler ausgebildet sein. In diesem Fall weist der Spannungsteiler 11 aktive Elemente auf, beispielsweise einen Operationsverstärker 13, der über nicht dargestellte Widerstände geeignet beschaltet ist.

Der Spannungsgenerator 10 generiert die Basisspannung U0 in der Regel hochgenau. Der Wert der Basisspannung U0 ist somit in weiten Grenzen unabhängig bzw. nahezu unabhängig von einer Versorgungsspannung des Spannungsgenerators 10 und auch unabhängig bzw. nahezu unabhängig von einer Umgebungstemperatur des Spannungsgenerators 10. Entsprechende Spannungsgeneratoren 10 sind Fachleuten allgemein bekannt. Beispielsweise kann entsprechend der Darstellung in den FIG 6 und 7 der Spannungsgenerator 10 als integrierter Schaltkreis des Typs REF 3225 der Firma Texas Instruments ausgebildet sein. In diesem Fall liegt die Basisspannung U0 bei einem Wert von 2,5 V. Auch andere Schaltkreise der Typenserie REF 32xx und entsprechende Schaltkreise anderer Hersteller können verwendet werden.

Der passive Spannungsteiler von FIG 6 kann fest eingestellt sein. Beispielsweise weist der passive Spannungsteiler von FIG 6 ausschließlich Widerstände 12 mit festen Widerstandswerten auf. In diesem Fall muss pro Referenzspannung U, die generiert werden soll, jeweils ein eigener passiver Spannungsteiler vorhanden sein. Alternativ ist es möglich, dass der passive Spannungsteiler von FIG 6 einstellbar ist. In diesem Fall kann beispielsweise entsprechend der Darstellung in FIG 8 mindestens einer der Widerstände 12 einstellbar sein. Die Einstellbarkeit kann manuell von außen oder elektronisch sein. Im Falle einer elektronischen Einstellbarkeit kann für eine sequenzielle Generierung mehrerer Referenzspannungen U unter Umständen ein und derselbe passive Spannungsteiler verwendet werden. Analoge Sachverhalte sind auch bei einem aktiven Spannungsteiler möglich.

Der Spannungsteiler 11 ist vorzugsweise als temperaturkompensierter Spannungsteiler ausgebildet. Dies kann beispielsweise bei einem passiven Spannungsteiler dadurch erreicht werden, dass die verwendeten Widerstände 12 gleichartige Temperaturcharakteristiken aufweisen. Analoge Ausgestaltungen (fest eingestellt oder einstellbar, Einstellbarkeit manuell von außen oder elektronisch) sind auch bei einer Ausgestaltung des Spannungsteilers 11 als aktiver Spannungsteiler möglich.

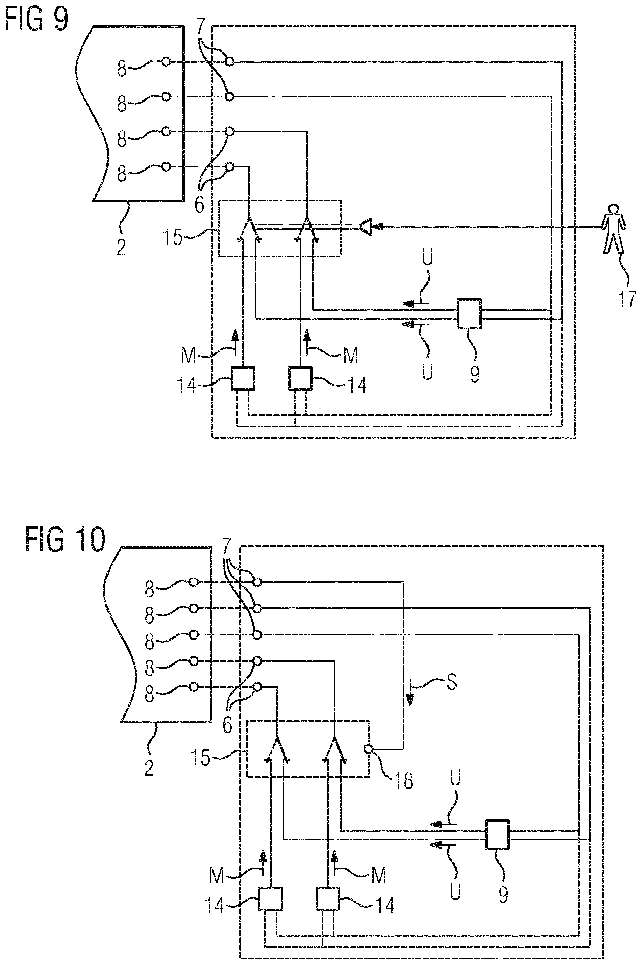

Die bisher erläuterten Ausgestaltungen gemäß den FIG 4 bis 8 stellen eine Minimalausgestaltung der Sonde 1 dar, bei welcher die Sonde 1 ausschließlich dazu verwendet wird, die Referenzspannungen U zur Verfügung zu stellen. Hingegen kann mit einer derartigen Sonde 1 keine echte Messung erfolgen. Es ist jedoch möglich, die Sonde 1 derart auszugestalten, dass sie sowohl die Referenzspannungen U zur Verfügung zu stellen kann als auch tatsächliche Messungen vornehmen kann. Dies wird nachfolgend in Verbindung mit FIG 9 und den darauf aufbauenden FIG näher erläutert.

Gemäß FIG 9 weist die Sonde 1 zusätzlich zur Referenzschaltung 9 eine Anzahl von Sensoreinrichtungen 14 auf. Minimal ist eine einzige Sensoreinrichtung 14 vorhanden. Es können aber auch mehrere Sensoreinrichtungen 14 vorhanden sein. Mittels der Sensoreinrichtungen 14 ist jeweils eine Zustandsgröße erfassbar, welche die Metallschmelze 4 aufweist. Beispielsweise können mittels entsprechender Sensoreinrichtungen 14 die Temperatur der Metallschmelze 4, der Sauerstoffgehalt der Metallschmelze 4, der Kohlenstoffgehalt der Metallschmelze 4 usw. erfasst werden. Entsprechende Sensoreinrichtungen 14 sind Fachleuten allgemein bekannt. Auch andere Größen können erfasst werden. In der Regel ist zumindest eine Sensoreinrichtung 14 zur Erfassung der Temperatur der Metallschmelze 4 vorhanden. Zusätzlich können Sensoreinrichtungen 14 zur Erfassung des Sauerstoffgehalts und/oder des Kohlenstoffgehalts der Metallschmelze 4 vorhanden sein. Die jeweilige Sensoreinrichtung 14 gibt ein Messsignal M aus, das mit der jeweiligen Zustandsgröße, wie sie von der jeweiligen Sensoreinrichtung 14 erfasst wurde, korrespondiert.

Die Sonde 1 weist weiterhin eine Umschalteinrichtung 15 auf. Die Umschalteinrichtung 15 befindet sich entweder in einem ersten Schaltzustand oder in einem zweiten Schaltzustand. Im ersten Schaltzustand - in FIG 9 in durchgezogenen Linien dargestellt - schaltet die Umschalteinrichtung 15 die Referenzschaltung 9 und damit die von der Referenzschaltung 9 abgegebenen Referenzspannungen U an die Signalkontakte 6 an. Im zweiten Schaltzustand - in FIG 9 in gestrichelten Linien dargestellt - schaltet die Umschalteinrichtung 15 anstelle der Referenzschaltung 9 die Sensoreinrichtungen 14 und damit die von den Sensoreinrichtungen 14 ausgegebenen Messsignale M an die Signalkontakte 6 an. Dadurch ist es möglich, zunächst (im ersten Schaltzustand der Umschalteinrichtung 15) die Referenzspannungen U an die Gegenkontakte 8 auszugeben und sodann (im zweiten Schaltzustand der Umschalteinrichtung 15) die Messsignale M an die Gegenkontakte 8 auszugeben.

In der Regel generiert die Referenzschaltung 9 für jeden Signalkontakt 6, über den im zweiten Schaltzustand der Umschalteinrichtung 15 ein Messsignal M ausgegeben wird, jeweils mindestens eine Referenzspannung U. Wenn also beispielsweise zwei Signalkontakte 6 vorhanden sind, über welche im zweiten Schaltzustand ein Messsignal M für die Temperatur der Metallschmelze 4 und ein Messsignal M für den Sauerstoffgehalt der Metallschmelze 4 ausgegeben werden, generiert die Referenzschaltung 9 für diese beiden Signalkontakte 6 jeweils eine eigene Referenzspannung U bzw. jeweils eine eigene Sequenz von Referenzspannungen U.

Zum Umschalten der Umschalteinrichtung 15 sind verschiedene Möglichkeiten gegeben.

So ist es beispielsweise entsprechend der Darstellung in FIG 9 möglich, dass die Umschalteinrichtung 15 eine Betätigungseinrichtung 16 aufweist, mittels derer die Umschalteinrichtung 15 von einer Bedienperson 17 manuell in den ersten und den zweiten Schaltzustand gebracht werden kann.

Alternativ ist es entsprechend der Darstellung in FIG 10 möglich, dass die Umschalteinrichtung 15 einen Signaleingang 18 aufweist. Der Signaleingang 18 kann in diesem Fall mit einem der übrigen Kontakte 7 verbunden sein. In diesem Fall ist es möglich, der Umschalteinrichtung 15 über den entsprechenden Gegenkontakt 8, den entsprechenden übrigen Kontakt 7 und den Signaleingang 18 ein Umschaltsignal S zuzuführen. Je nachdem, welchen Wert das Umschaltsignal S aufweist, nimmt die Umschalteinrichtung 15 den ersten oder den zweiten Schaltzustand an. Die Umschalteinrichtung 15 kann dadurch ferngesteuert gezielt vom ersten in den zweiten Schaltzustand und umgekehrt auch gezielt vom zweiten in den ersten Schaltzustand gebracht werden.

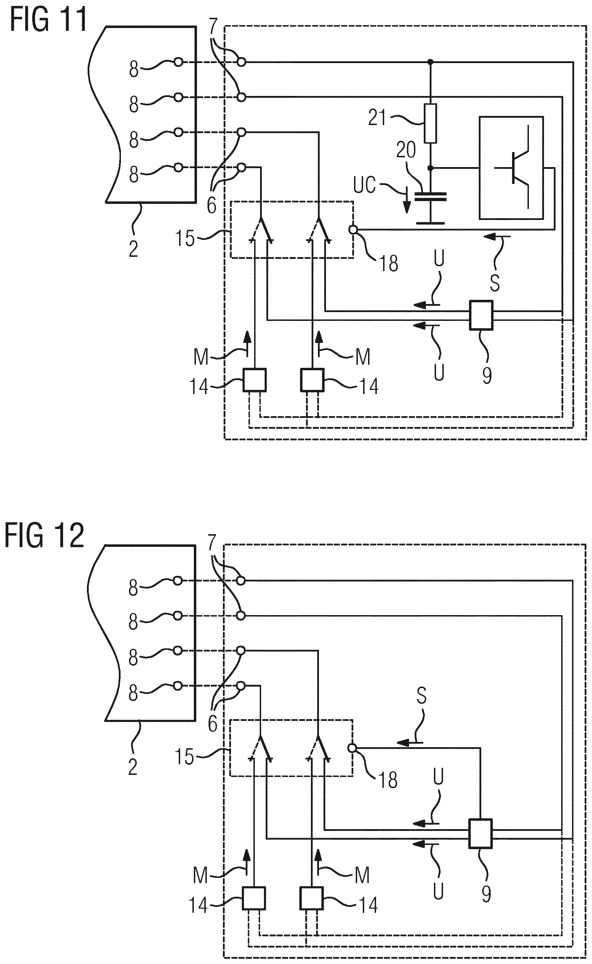

Alternativ ist es entsprechend der Darstellung in FIG 11 möglich, dass die Umschalteinrichtung 15 derart ausgebildet ist, dass sie beim Aufstecken der Sonde 1 auf die Lanze 2 stets zunächst den ersten Schaltzustand annimmt. Der Übergang in den zweiten Schaltzustand kann in diesem Fall auf verschiedene Art und Weise bewerkstelligt werden.

Beispielsweise kann der Umschalteinrichtung 15 eine Zusatzschaltung 19 zugeordnet sein, welche nach Ablauf einer vorbestimmten Zeitspanne eine Umschaltung der Umschalteinrichtung 15 vom ersten in den zweiten Schaltzustand bewirkt. Die Zusatzschaltung 19 kann beispielsweise als Timer ausgebildet sein, also als Zähler, der getaktet inkrementiert wird und bei einem bestimmten Zählerstand ein binäres Signal ausgibt. Alternativ kann die Zusatzschaltung 19 - rein beispielhaft - entsprechend der Darstellung in FIG 11 einen Kondensator 20 und einen vorgeschalteten Vorwiderstand 21 aufweisen, so dass der Kondensator 20 ab dem Aufstecken der Sonde 1 auf die Lanze 2 über eine der Sonde 1 zugeführte Versorgungsspannung geladen wird. Die über dem Kondensator 20 abfallende Kondensatorspannung UC kann in diesem Fall einer Schalteinrichtung 22 zugeführt werden, welche bei Erreichen eines kritischen Wertes die Schalteinrichtung 22 betätigt, die das Umschaltsignal S abgibt. Das Betätigen der Schalteinrichtung 22 bewirkt in diesem Fall, dass das Umschaltsignal S seinen Wert wechselt und dadurch die Umschalteinrichtung 15 vom ersten in den zweiten Schaltzustand überführt wird. Die Schalteinrichtung 22 kann beispielsweise als entsprechend beschalteter Transistor ausgebildet sein. Es sind aber auch andere Ausgestaltungen möglich.

Alternativ ist es entsprechend der Darstellung in FIG 12 möglich, dass die Referenzschaltung 9 das Umschaltsignal S ausgibt. Diese Ausgestaltung ist insbesondere dann sinnvoll, wenn die Referenzschaltung 9 nicht nur eine einzelne Referenzspannung U ausgibt, sondern eine Sequenz von Referenzspannungen U. In diesem Fall hält die Referenzschaltung 9 das Umschaltsignal S auf dem Wert für den ersten Schaltzustand, solange die Sequenz von Referenzspannungen U ausgegeben wird. Nach Beenden des Ausgebens der Sequenz von Referenzspannungen ändert die Referenzschaltung 9 den Wert des Umschaltsignals S auf den Wert für den zweiten Schaltzustand.

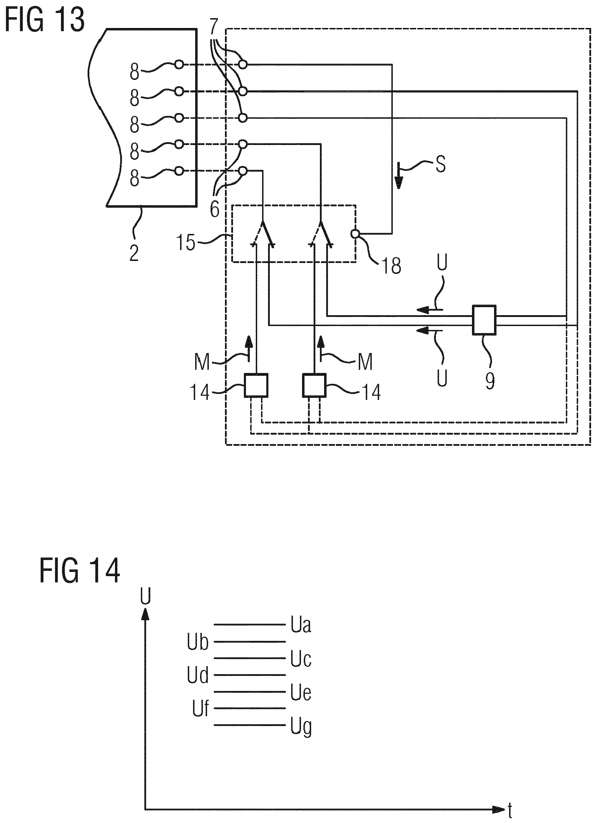

Alternativ ist es entsprechend der Darstellung in FIG 13 möglich, dass das Umschaltsignal S der Umschalteinrichtung 15 - analog zu FIG 10 - über einen der übrigen Kontakte 7 vorgegeben wird. Im Falle der Ausgestaltung von FIG 13 ist die Umschalteinrichtung 15 jedoch derart ausgebildet, dass sie zwar einmal vom ersten in den zweiten Schaltzustand überführt werden kann, ein späterer Wechsel zurück in den ersten Schaltzustand jedoch nicht mehr möglich ist. Die Vorgabe des Umschaltsignals S zum Umschalten in den zweiten Schaltzustand führt also in diesem Fall zu einem irreversiblen Übergang in den zweiten Schaltzustand.

Es ist möglich, die Zusatzschaltung 19, welche die Umschalteinrichtung 15 ansteuert, aus diskreten Bauelementen - beispielsweise dem Kondensator 20, dem Vorwiderstand 21 und der Schalteinrichtung 22 - realisiert ist. Alternativ ist es möglich, die Zusatzschaltung 19 mit integrierten Schaltkreisen zu realisieren. Die Umschalteinrichtung 15 selbst kann als elektromechanische Umschalteinrichtung oder als elektronische Umschalteinrichtung ausgebildet ist. Schaltelemente der Umschalteinrichtung 15 können im erstgenannten Fall beispielsweise als Relais und im letztgenannten Fall beispielsweise als MOSFETs ausgebildet sein.

Wenn die Sonde 1 zusätzlich zur Referenzschaltung 9 auch die Sensoreinrichtungen 14 und die Umschalteinrichtung 15 aufweist, kann die Sonde 1 weiterhin entsprechend der Darstellung in FIG 1 einen Hohlraum 23 aufweisen. In diesem Fall ist es möglich, mittels des Hohlraums 23 aus der Metallschmelze 4 eine Probe zu entnehmen. Die entnommene Probe kann später in einem Labor analysiert werden. Unabhängig davon, ob die Sonde 1 zusätzlich zur Referenzschaltung 9 auch die Sensoreinrichtungen 14 und die Umschalteinrichtung 15 aufweist oder nicht, kann die Mantelfläche 24 der Sonde 1 jedoch - so wie im Stand der Technik auch - aus Karton bestehen, insbesondere aus gewickeltem Karton.

Aus den obenstehenden Ausführungen ist ersichtlich, dass es verschiedene Typen von Sonden 1 geben kann, die zwar alle die Referenzspannungen U ausgeben und damit als erfindungsgemäße Sonden 1 ausgebildet sind, sich aber in anderer Hinsicht voneinander unterscheiden. Beispielsweise können folgende Sonden 1 vorhanden sein:
- Eine Sonde 1, die als reine Referenzsonde ausgebildet ist, welche keine tatsächliche Messungen vornehmen kann. Diese Sonde wird nachfolgend als Referenzsonde bezeichnet und mit dem Bezugszeichen 1a versehen.
- Eine Sonde 1, mit der die Temperatur der Metallschmelze 4 erfasst werden kann. Diese Sonde wird nachfolgend als T-Sonde bezeichnet und mit dem Bezugszeichen 1b versehen.
- Eine Sonde 1, mit der die Temperatur der Metallschmelze 4 und der Sauerstoffgehalt der Metallschmelze 4 erfasst werden können. Diese Sonde wird nachfolgend als TO-Sonde bezeichnet und mit dem Bezugszeichen 1c versehen.
- Eine Sonde 1, mit der die Temperatur der Metallschmelze 4 und der Kohlenstoffgehalt der Metallschmelze 4 erfasst werden können. Diese Sonde wird nachfolgend als TC-Sonde bezeichnet und mit dem Bezugszeichen 1d versehen.
- Eine Sonde 1, mit der die Temperatur der Metallschmelze 4 erfasst werden kann und zusätzlich eine Probe der Metallschmelze 4 gezogen werden kann. Diese Sonde wird nachfolgend als TS-Sonde bezeichnet und mit dem Bezugszeichen 1e versehen.
- Eine Sonde 1, mit der die Temperatur der Metallschmelze 4 und der Sauerstoffgehalt der Metallschmelze 4 erfasst werden können und zusätzlich eine Probe der Metallschmelze 4 gezogen werden kann. Diese Sonde wird nachfolgend als TSO-Sonde bezeichnet und mit dem Bezugszeichen 1f versehen.
- Eine Sonde 1, mit der die Temperatur der Metallschmelze 4 und der Kohlenstoffgehalt der Metallschmelze 4 erfasst werden können und zusätzlich eine Probe der Metallschmelze 4 gezogen werden kann. Diese Sonde wird nachfolgend als TSC-Sonde bezeichnet und mit dem Bezugszeichen 1g versehen.

Alle diese Sonden 1, also die Sonden 1a bis 1g, können den gleichen mechanischen Aufbau aufweisen. Weiterhin wird bei allen Sonden 1 mit Ausnahme der Referenzsonde 1a die Temperatur der Metallschmelze 4 und das zugehörige Messsignal M demselben Gegenkontakt 8 zugeführt. Die zugehörige Sensoreinrichtung 14 ist also eine Temperaturmesseinrichtung. Derjenige Signalkontakt 6, über den das Messsignal M für die Temperatur der Metallschmelze 4 dem erwähnten Gegenkontakt 8 zugeführt wird, befindet sich also nach dem Aufstecken der jeweiligen Sonde 1 auf die Lanze 2 immer an der Stelle, dass er diesen Gegenkontakt 8 kontaktiert. Der entsprechende Signalkontakt 6 der Sonden 1 wird nachfolgend als Sonderkontakt bezeichnet und mit dem Bezugszeichen 6' versehen. Mit der Bezeichnung als Sonderkontakt und der Vergabe des Bezugszeichen 6' soll jedoch keine spezielle Funktion verbunden sein, sondern nur eine sprachliche Individualisierung zur Unterscheidung von den anderen Signalkontakten 6 der Sonden 1.

Allgemein gesprochen unterscheiden sich die Sonden 1 also durch mindestens eines der nachfolgenden Kriterien voneinander:
- Die Antwort auf die Frage, ob und gegebenenfalls in welchem Umfang die Sonden 1 weitere Signalkontakte 6 aufweisen, über die sie jeweils eine Anzahl von Referenzspannungen U an den jeweiligen Gegenkontakt 8 ausgeben. Insbesondere ist es bei der Referenzsonde 1a sinnvoll, über zwei Signalkontakte 6, also zusätzlich zum Sonderkontakt 6' über einen weiteren Signalkontakt 6, Referenzspannungen U auszugeben. Gleiches gilt für die TO-Sonde 1c, die TC-Sonde 1d, die TSO-Sonde 1f und die TSC-Sonde 1g. Für die T-Sonde 1b und die TS-Sonde 1e ist es hingegen ausreichend, ausschließlich über den Sonderkontakt 6' eine Referenzspannung U bzw. eine Sequenz von Referenzspannungen U abzugeben. Falls Sonden 1 existieren sollten, mittels der mehr als zwei Messsignale M erfasst werden können, kann es bei der Referenzsonde 1a auch sinnvoll sein, ebenso über mehr als zwei Signalkontakte entsprechende Referenzspannungen U auszugeben.
- Die Antwort auf die Frage, ob die Sonden 1 eine Sensoreinrichtung 14 und eine Umschalteinrichtung 15 aufweisen, so dass sie über den Sonderkontakt 6' alternativ zu der Anzahl von Referenzspannungen U ein Messsignal M ausgeben können. Insbesondere kann über den Sonderkontakt 6' der Referenzsonde 1a kein Messsignal M ausgegeben werden, während dies bei den anderen Sonden 1b bis 1g der Fall ist.
- Die Antwort auf die Frage, ob und gegebenenfalls in welchem Umfang die Sonden 1 weitere Sensoreinrichtungen 14 aufweisen und über die Umschalteinrichtung 15 alternativ zu der Anzahl von Referenzspannungen U weitere Messsignale M an vom Sonderkontakt 6' verschiedene Signalkontakte 6 ausgeben können. Insbesondere kann mittels der Referenzsonde 1a kein Messsignal M ausgegeben werden. Andererseits kann mittels der T-Sonde 1b und die TS-Sonde 1e nur das Messsignal M für die Temperatur der Metallschmelze 4 ausgegeben werden. Hingegen kann mittels der TO-Sonde 1c, mittels der TC-Sonde 1d, mittels der TSO-Sonde 1f und mittels der TSC-Sonde 1g zusätzlich zum Messsignal M für die Temperatur der Metallschmelze 4 ein weiteres Messsignal M für den Sauerstoffgehalt bzw. den Kohlenstoffgehalt der Metallschmelze 4 ausgegeben werden. Es ist auch eine Sonde 1 denkbar, bei der zusätzlich zum Messsignal M für die Temperatur der Metallschmelze 4 mehrere weitere Messsignale M, beispielsweise sowohl für den Kohlenstoffgehalt als auch für den Sauerstoffgehalt, erfasst werden können.
- Die Antwort auf die Frage, welche Sensoreinrichtungen 14 die Sonden 1 gegebenenfalls aufweisen und mit welchen Zustandsgrößen der Metallschmelze 4 die zugehörigen Messsignale M korrespondieren. Beispielsweise können verschiedenartige Temperatursensoren vorhanden sein, deren Messsignale M bei gleicher Temperatur der Metallschmelze 4 voneinander verschiedene Messwerte M abgeben. Auch kann die Zustandsgröße von Sonde 1 zu Sonde 1 verschieden sein. Beispielsweise können bei der TO-Sonde 1c, der TC-Sonde 1d, der TSO-Sonde 1f und der TSC-Sonde 1g die Messsignale M für den Sauerstoffgehalt bzw. den Kohlenstoffgehalt der Metallschmelze 4 zwar jeweils demselben Gegenkontakt 8 der Lanze 2 zugeführt werden. Die Bedeutung dieses Messsignals M unterscheidet sich aber je nachdem, ob es sich bei der betreffenden Sonde 1 um die TO-Sonde 1c oder die TSO-Sonde 1f einerseits oder die TC-Sonde 1d oder die TSC-Sonde 1g andererseits handelt. In den beiden erstgenannten Fällen ist das Messsignal M für den Sauerstoffgehalt der Metallschmelze 4 charakteristisch, in den beiden letztgenannten Fällen für den Kohlenstoffgehalt der Metallschmelze 4.
- Die Antwort auf die Frage, ob die Sonde 1 den Hohlraum 23 aufweist oder nicht, ob also mittels der entsprechenden Sonde 1 aus der Metallschmelze 4 eine Probe entnommen werden kann oder nicht. Insbesondere ist die Entnahme einer Probe möglich bei der TS-Sonde 1e, der TSO-Sonde 1f und der TSC-Sonde 1g, während sie bei der Referenzsonde 1a, der T-Sonde 1b, der TO-Sonde 1c und der TC-Sonde 1d nicht möglich ist.

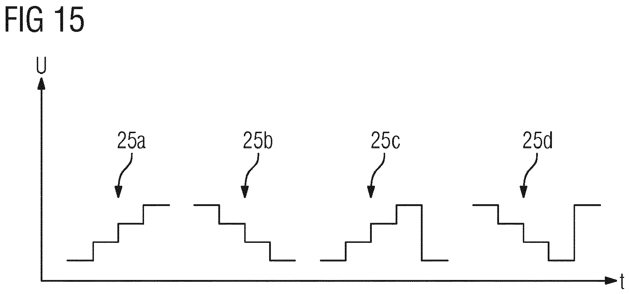

Es ist möglich, dass bei allen Sonden 1 die ausgegebenen Referenzspannungen U bzw. Sequenzen von Referenzspannungen U die gleichen sind. Vorzugsweise unterscheiden sich die Sonden 1 jedoch dahingehend, dass der Wert der Referenzspannung U und/oder die Abfolge der Referenzspannungen U, welche die Sonden 1 über den Sonderkontakt 6' ausgeben, voneinander verschieden sind. Wenn die Sonden 1 über den Sonderkontakt 6' jeweils eine einzelne Referenzspannung U ausgeben, kann beispielsweise je nachdem, ob es sich bei der Sonde 1 um die Sonde 1a, die Sonde 1b usw. handelt, entsprechend der Darstellung in FIG 14 die Referenzspannung U jeweils einen anderen Wert aufweisen. Die Referenzspannungen U sind in FIG 14 jeweils mit einem kleinen Buchstaben a bis g ergänzt, um anzudeuten, für welche Sonde 1a bis 1g die jeweilige Referenzspannung U gilt. Wenn die Sonden 1 über den Sonderkontakt 6' jeweils eine Sequenz von Referenzspannungen U ausgeben, kann beispielsweise je nachdem, ob es sich bei der Sonde 1 um die Sonde 1a, die Sonde 1b usw. handelt, die entsprechende Sequenz von Referenzspannungen U jeweils individuell definiert sein. Beispielsweise ist es entsprechend der Darstellung in FIG 15 möglich,
- für die Referenzsonde 1a eine Sequenz 25a von aufeinander folgenden, streng monoton steigenden Referenzspannungen U ausgegeben werden,
- für die T-Sonde 1b eine Sequenz 25b von aufeinanderfolgenden, streng monoton fallenden Referenzspannungen U,
- für die TO-Sonde 1c eine Sequenz 25c von aufeinanderfolgenden, streng monoton steigenden Referenzspannungen U, die am Ende der Folge durch einen deutlich niedrigeren Wert abgeschlossen wird, und
- für die TC-Sonde 1d eine Sequenz 25d von aufeinanderfolgenden, streng monoton fallenden Referenzspannungen U, die am Ende der Folge durch einen deutlich höheren Wert abgeschlossen wird.

Wenn nur diese vier Typen von Sonden 1 vorhanden sind, also nur die Referenzsonde 1a, die T-Sonde 1b, die TO-Sonde 1c und die TC-Sonde 1d, können hierdurch diese vier Typen von Sonden 1 eindeutig erkannt und voneinander unterschieden werden. In ähnlicher Weise kann aber auch für die TS-Sonde 1e, für die TSO-Sonde 1f und für die TSC-Sonde 1g jeweils eine entsprechende Sequenz von Referenzspannungen U ausgegeben werden, so dass auch bei mehr als vier Typen von Sonden 1 der Typ der jeweiligen Sonde 1 eindeutig erkennbar ist.

Die vorliegende Erfindung weist viele Vorteile auf. Durch die Kombination der Referenzschaltung 9, der Umschalteinrichtung 15 und der Sensoreinrichtungen 14 ist es möglich, vor jedem Messvorgang, also vor jeder Verwendung einer Sonde 1, zuverlässig den jeweiligen Messkreis zu überprüfen und gegebenenfalls sogar einen Offset zur Kompensation eines Messfehlers zu ermitteln. Messfehler können erkannt und in vielen Fällen sogar kompensiert werden. Die jeweilige Referenzspannung U bzw. die jeweilige Sequenz von Referenzspannungen U kann geeignet bestimmt werden, so dass sie in dem in der Praxis auftretenden Messbereich liegt bzw. diesen abdeckt. Beispielsweise kann für eine Sensoreinrichtung 14, mittels derer die Temperatur der Metallschmelze 4 erfasst werden soll, im Fall einer Stahlschmelze eine Referenzspannung U gewählt werden, die einer Temperatur im Bereich zwischen 1600 °C und 1700 °C entspricht. Es ist weiterhin problemlos möglich, die Referenzspannungen U auf Seiten einer Auswertungseinrichtung als solche zu erkennen. Denn da bekannt ist, zu welchen Zeiten die jeweilige Sonde 1 mit der Lanze 2 verbunden wird, ist auch bekannt, dass zunächst die Referenzspannungen U ausgegeben werden und erst danach eventuelle Messsignale M.

Obwohl die Erfindung im Detail durch das bevorzugte Ausführungsbeispiel näher illustriert und beschrieben wurde, so ist die Erfindung nicht durch die offenbarten Beispiele eingeschränkt und andere Varianten können vom Fachmann hieraus abgeleitet werden, ohne den Schutzumfang der Erfindung zu verlassen.

### Bezugszeichenliste

- 1, 1a bis 1g: Sonden
- 2: Lanze
- 3: metallurgisches Gefäß
- 4: Metallschmelze
- 5: Sondenachse
- 6: Signalkontakte
- 6': Sonderkontakt
- 7: übrige Kontakte
- 8: Gegenkontakte
- 9: Referenzschaltung
- 10: Spannungsgenerator
- 11: Spannungsteiler
- 12: Widerstände
- 13: Operationsverstärker
- 14: Sensoreinrichtungen
- 15: Umschalteinrichtung
- 16: Betätigungseinrichtung
- 17: Bedienperson
- 18: Signaleingang
- 19: Zusatzschaltung
- 20: Kondensator
- 21: Vorwiderstand
- 22: Schalteinrichtung
- 23: Hohlraum
- 24: Mantelfläche
- 25a bis 25d: Sequenzen

- d: Durchmesser
- M: Messsignale
- l: Länge
- S: Umschaltsignal
- t: Zeit
- U, Ua bis Ug: Referenzspannungen
- U0: Basisspannung
- UC: Kondensatorspannung

## Patentansprüche

1. Sonde für eine Lanze (2) eines metallurgischen Gefäßes (3),
- wobei die Sonde im wesentlichen bezüglich einer Sondenachse (5) rotationssymmetrisch ist, insbesondere zylindrisch ist,
- wobei die Sonde durch Stecken in Richtung der Sondenachse (5) auf die Lanze (2) des metallurgischen Gefäßes (3) aufsteckbar ist,
- wobei die Sonde eine Anzahl von Signalkontakten (6) und eine Anzahl von übrigen Kontakten (7) aufweist, die beim Aufstecken der Sonde auf die Lanze (2) mit jeweiligen Gegenkontakten (8) der Lanze (2) kontaktiert werden,
**dadurch gekennzeichnet,**
- **dass** die Sonde eine Referenzschaltung (9) aufweist, die derart ausgebildet ist, dass sie pro Signalkontakt (6) eine Anzahl von Referenzspannungen (U) generiert und über den jeweiligen Signalkontakt (6) an den jeweiligen Gegenkontakt (8) ausgibt, und
- **dass**, bezogen auf den jeweiligen Signalkontakt (6), entweder die Anzahl von Referenzspannungen (U) gleich eins ist oder die Referenzspannungen (U) eine Sequenz von nacheinander ausgegebenen Referenzspannungen (U) bilden.

2. Sonde nach Anspruch 1,
**dadurch gekennzeichnet,**
**dass** die Referenzschaltung (9) einen Spannungsgenerator (10) aufweist, der eine Basisspannung (U0) generiert, und dass die Referenzspannungen (U) aus der Basisspannung (U0) über Spannungsteiler (11) generiert werden.

3. Sonde nach Anspruch 2,
**dadurch gekennzeichnet,**
**dass** die Spannungsteiler (11) als passive Spannungsteiler oder als aktive Spannungsteiler ausgebildet sind.

4. Sonde nach Anspruch 2 oder 3,
**dadurch gekennzeichnet,**
**dass** die Spannungsteiler (11) fest eingestellt oder einstellbar sind.

5. Sonde nach Anspruch 2, 3 oder 4,
**dadurch gekennzeichnet,**
**dass** die Spannungsteiler (11) als temperaturkompensierte Spannungsteiler ausgebildet sind.

6. Sonde nach einem der obigen Ansprüche,
**dadurch gekennzeichnet,**
- **dass** die Sonde eine Anzahl von Sensoreinrichtungen (14) aufweist, mittels derer jeweils eine Zustandsgröße einer in dem metallurgischen Gefäß (3) befindlichen Metallschmelze (4) erfassbar ist und ein mit der jeweiligen Zustandsgröße korrespondierendes Messsignal (M) ausgebbar ist, und
- **dass** die Sonde eine Umschalteinrichtung (15) aufweist, welche in einem ersten Schaltzustand die Referenzschaltung (9) und in einem zweiten Schaltzustand anstelle der Referenzschaltung (9) die Sensoreinrichtungen (14) an die Signalkontakte (6) anschaltet.

7. Sonde nach Anspruch 6,
**dadurch gekennzeichnet,**
- **dass** die Umschalteinrichtung (15) eine Betätigungseinrichtung (16) aufweist, mittels derer die Umschalteinrichtung (15) von einer Bedienperson (17) manuell in den ersten und den zweiten Schaltzustand gebracht werden kann, oder
- **dass** die Umschalteinrichtung (15) einen Signaleingang (18) aufweist, der mit einem der übrigen Kontakte (7) verbunden ist, und dass die Umschalteinrichtung (15) mittels eines diesem übrigen Kontakt (7) über den korrespondierenden Gegenkontakt (8) zugeführten Umschaltsignals (S) in den ersten und den zweiten Schaltzustand gebracht werden kann, oder
- **dass** die Umschalteinrichtung (15) derart ausgebildet ist, dass sie beim Aufstecken der Sonde auf die Lanze (2) stets zunächst den ersten Schaltzustand annimmt und in den zweiten Schaltzustand übergeht
-- nach Ablauf einer vorbestimmten Zeitspanne,
-- im Falle des Ausgebens einer Sequenz von Referenzspannungen (U) nach Beenden des Ausgebens der Sequenz von Referenzspannungen (U) oder
-- aufgrund der Vorgabe eines Umschaltsignals (S) über einen der übrigen Kontakte (7).

8. Sonde nach Anspruch 6 oder 7,
**dadurch gekennzeichnet,**
**dass** die Sonde eine Zusatzschaltung (19) aufweist, welche die Umschalteinrichtung (15) ansteuert und dass die Zusatzschaltung (19) aus diskreten Bauelementen oder mit integrierten Schaltkreisen realisiert ist.

9. Sonde nach Anspruch 6, 7 oder 8,
**dadurch gekennzeichnet,**
**dass** die Umschalteinrichtung (15) als elektromechanische Umschalteinrichtung oder als elektronische Umschalteinrichtung ausgebildet ist.

10. Sonde nach einem der Ansprüche 6 bis 9,
**dadurch gekennzeichnet,**
**dass** die Sonde einen Hohlraum (23) aufweist, mittels dessen aus einer in dem metallurgischen Gefäß (3) befindlichen Metallschmelze (4) eine Probe entnommen werden kann.

11. Sonde nach einem der obigen Ansprüche,
**dadurch gekennzeichnet,**
**dass** die Mantelfläche (24) der Sonde aus Karton besteht, insbesondere aus gewickeltem Karton.

12. Gruppe von Sonden (1) nach einem der Ansprüche 1 bis 11,
- wobei die Sonden (1) den gleichen mechanischen Aufbau aufweisen,
- wobei einer der Signalkontakte (6) der Sonden (1) ein Sonderkontakt (6') ist,
- wobei die Sonderkontakte (6') der Sonden (1) beim Aufstecken der jeweiligen Sonde (1) in die Lanze (2) denselben Gegenkontakt (8) kontaktieren,
**dadurch gekennzeichnet,**
- **dass** die Sonden (1) sich dadurch unterscheiden,
-- ob und gegebenenfalls in welchem Umfang sie weitere Signalkontakte (6) aufweisen, über die sie jeweils eine Anzahl von Referenzspannungen (U) an den jeweiligen Gegenkontakt (8) ausgeben, und/oder
-- ob sie eine Sensoreinrichtung (14) und eine Umschalteinrichtung (15) aufweisen, so dass sie über den Sonderkontakt (6') alternativ zu der Anzahl von Referenzspannungen (U) ein Messsignal (M) ausgeben können, und/oder
-- ob und gegebenenfalls in welchem Umfang sie weitere Sensoreinrichtungen (14) aufweisen und über die Umschalteinrichtung (15) alternativ zu der Anzahl von Referenzspannungen (U) weitere Messsignale (M) ausgeben können und/oder
-- gegebenenfalls welche Sensoreinrichtungen (14) sie aufweisen und mit welchen Zustandsgrößen der in dem metallurgischen Gefäß (3) befindlichen Metallschmelze (4) die zugehörigen Messsignale (M) korrespondieren und/oder
-- ob die Sonde (1) einen Hohlraum (23) aufweist, mittels dessen aus der in dem metallurgischen Gefäß (3) befindlichen Metallschmelze (4) eine Probe entnommen werden kann, und
- **dass** die Sonden (1) sich weiterhin dadurch unterscheiden, dass der Wert der Referenzspannung (U) und/oder die Abfolge der Referenzspannungen (U), welche die Sonden (1) über den Sonderkontakt (6') ausgeben, voneinander verschieden sind.

13. Gruppe von Sonden (1) nach Anspruch 12,
**dadurch gekennzeichnet,**
**dass** bei allen Sonden (1), bei denen über den Sonderkontakt (6') ein Messsignal (M) übertragen werden kann, die zugehörige Sensoreinrichtung (14) eine Temperaturmesseinrichtung ist.
